# EUROPEAN PATENT SPECIFICATION

(11) **EP 0 224 505 B1**
(45) Date of publication and mention of the grant of the patent: **01.06.1994**
(21) Application number: 86902984.3
(22) Date of filing: 16.04.1986
(51) Int. Cl.: A61F 13/00

(54) **ISOLATOR FOR USE IN SURGERY**
ISOLATOR FÜR CHIRURGIE
ISOLATEUR UTILISE EN CHIRURGIE

(30) Priority: 23.04.1985 US 726088
(43) Date of publication of application: 10.06.1987
(73) Proprietor: LONE STAR MEDICAL PRODUCTS, INC., Houston, TX 77054 (US)
(72) Inventor: SCOTT, Frank, B., Houston, TX 77030 (US); FIELDS, Charles, J., Houston, TX 77035 (US); FOWLER, James, M., Jr., Houston, TX 77092 (US)
(74) Representative: Allam, Peter Clerk
(86) International application number: US8600807
(87) International publication number: WO8606272

(56) References cited:
- US-A- 3 850 172
- US-A- 3 907 389
- US-A- 4 026 286
- US-A- 4 161 172
- US-A- 4 275 719
- US-A- 4 328 793
- US-A- 4 367 728
- No relevant documents have been disclosed.

## Description

This invention relates to an isolator for providing a contaminant-free atmosphere in which a surgical procedure can be performed or equipment sensitive to environmental contamination can be assembled.

A large amount of effort is spent in the avoidance of contamination of surgical wounds by disease organisms in the operating room. All instruments and dry goods coming into contact with the surgical field are sterilized, either in an autoclave or with chemicals. Chemicals are used to sterilize the patient's skin in the area of the surgery. The surgical team scrub their hands and arms for at least five minutes after which hands and arms are bathed in alcohol. Sterilized gowns, caps, and masks that filter the team's exhaled air are worn by the surgical team along with sterilized gloves that cover their hands. Thereafter, the surgical team avoids contact with non-sterilized objects. Further, the air in the operating room is constantly changed and filtered.

Even with all this preparation and attention to sterilization, a significant percentage of supposedly clean operations result in wound infections, which means that operating aseptically on man remains only a concept and not an accomplished procedure.

One of the problems is that disease organisms are ubiquitous in operating room air, on the patient's skin, in his gastrointestinal tract, and in the exhalations of all persons in the room. Also, the individuals of the surgical team shed disease organisms as they move around the operating room during the surgery. For example, it has been determined that 30,000 to 60,000 particles are shed each minute from each person in the operating room. Foreign particles that have been shown to cause granuloma, a type of infection, include lint, wood fibers, talc dust and related agents. These particles come from drapes, gloves, wrapping materials and other items found inside the operating room (OR). Bacterial contaminants are released into the atmosphere from the skin and hair, by breathing and passing gas, and from the urinary tracts of the surgical team and the patient.

There are certain types of surgery that experience a higher than normal rate of infection. Surgeries involving the implantation of a prosthetic device or artificial organ is a common example. The dose of bacteria necessary to invade the wound and cause an infection is reduced when foreign matter, such as an implanted device, is introduced into the body. Repeated experimental and clinical studies have proven that the mere presence of a foreign body can seriously impede the human body's immune system. Over 50,000 bacteria may be required to cause a surgical wound infection in normal operations whereas only 100 bacteria can cause infection when an implant device, even though inert, is introduced. In some implant surgeries, it has been theorized that a single bacterium may be all that is necessary to cause a deep wound infection.

Certainly many wound infections can be attributed to endogenous (patient) causes. It is generally agreed, though, that airborne contamination during surgery contributes in some degree to the number of infections. Indeed, there is much empirical evidence and many major comprehensive studies that suggest it as the prime contributor. The complications that develop from wound infection can be very serious and it is a continuing problem that plagues the operating community as well as the patient.

To assist in maintaining a contaminant-free atmosphere in which a surgical procedure can be performed or equipment sensitive to environmental contamination can be assembled, isolators have been developed in which the contaminant-free atmosphere can be maintained as a preventative measure against surgical wound infections.

The first surgical isolators were developed for use in gnotobiotics where germ-free laboratory animals were obtained by delivering such animals from their parents by Caesarean section directly into an aseptic environment. Later a plastic isolator for use on humans was designed by Levenson, et al and described in an article entitled "A Plastic Isolator for Operating in a Sterile Environment" American Journal of Surgery, 104, 891-899, 1962. A subsequent isolator was developed by McLauchlan, et al and described in an article entitled "The Surgical Isolator", British Medical Journal, 1(903): 322-4, 23 February, 1974. Both of these isolators are discussed in "Air Contamination Control in Hospitals" by Joseph R. Luciano, Copyright 1977, Plenum Press, New York, pages 355-359.

Both the Levenson and McLauchlan isolators included bags of thin, flexible, plastic material inflated with sterile air. Both have jackets or sleeves that extend into the bag and that cover the surgeon and his assistant's arms during the surgery. The jackets and sleeves are closed at their ends by the gloves that the surgeons wear. This makes it very difficult for the surgeons to change gloves during the surgery should one of the gloves tear. Also, should it be necessary to remove the isolator and complete the surgery without it, the surgeon would have to be regloved before he could proceed. Another disadvantage of this arrangement, if that since the gloves are attached to their ends, the sleeves or jackets extend into the work area of the isolator to the same extent as the arms of the surgical team and tends to partially obscure the vision of the surgeon, as well as just being somewhat in the way of the surgical team as it works.

The isolators of both Levenson and McLauchlan had clear plastic in the top through which the surgical team could view the surgical field, but the plastic was flexible and would change shapes depending upon the air pressure in the isolator and would tend to balloon away from the surgical field.

Other forms of isolator have been proposed. U.S. Patent US-A-4,275,719 (Mayer) shows an enclosure supported on a frame into which the patient is placed for surgery. The enclosure has sterilized air pumped into it but is not itself sealed. The patient on whom surgery is to be performed is wrapped in a closely fitting sheet or film through which sterile air is circulated. However, the surgery is not performed within the sheet. It has to be slit at the point of surgery to allow access to the patient's body. The surgeon operates within the larger, non-sealed, enclosure.

U.S. patent US-A-4,026,286 (Trexler) shows a larger sealed enclosure in which a flexible clear plastic sheet is supported on a rectangular frame in a box-like structure. An access port for the passage of objects is provided at one end of the enclosure with provision made for air pressure within the enclosure acting to prevent entry of contaminants. One side of the enclosure is formed with integral gloves for manipulating objects in the enclosure. The objects being manipulated are viewed through the side of the flexible sheet which, although subject to the internal air pressure, is primarily supported rather loosely by the frame.

U.S. Patent US-A-4,367,728 (Mutke) discloses an isolator comprising an inflated sack or bag of a flexible clear plastic. However, the sack is not self-supporting under inflation but has additional supports attached to the top of the sack. To improve visibility into the area of surgery a window is provided at the required position by clamping a small portion of the upper part of the sack over a ring. Gloves for internal manipulation are also provided in the upper part of and other accessories are fitting to this upper portion also. The upper portion including the small window is liable to move in response to any forces acting on it as a result of the procedures being performed.

There will be described hereinafter embodiments of an isolator in accord with this invention which includes a tent or bag (also referred to as a bubble) of flexible, impervious material for placing on the area of the patient where the surgery is to be done. Into the bag is pumping a continuous stream of filtered air that flows from one side of the bubble across the surgical wound to exhaust ports on the opposite sides of the bag to maintain the bag inflated at a pressure above atmospheric and to constantly change the air in the isolator. The bag is supported solely by the pressure established in it.

More particularly there will be described an isolator comprising an inflatable bag having side and end walls of flexible material and an upper side, a portion of which is of flexible material and a portion of which is of a relatively stiff, optically transparent material, that is designed so that when the bag is inflated, it will assume a position a predetermined distance above the surgical field and remain substantially in that position throughout the operation giving the surgeon and assistants a clear and unlimited view of the surgical field and ample room inside the bag in which to work. To this end, the surgical isolator to be described comprises an inflatable bag of flexible material that is impervious to disease organisms and that includes a section in the upper wall of relatively stiff, optically transparent, material that will assume a position when the bag is inflated that is substantially straight along the longitudinal axis of the bag and slightly convex or V-shaped along the transverse axis of the bag to provide a window through which the surgeon has a clear view of the surgical field.

It will be further shown how such an isolator can be arranged and used so that the instruments and other material used during the surgery are moved into and out of the bag through a door in the side where exhaust ports are located so the air is always moving in a direction to keep any contaminants outside the bag from entering the bag while the door is open.

In one embodiment of an isolator according to this invention the instruments and other material are moved into and out of the bag through an air lock, the doors of which have exhaust ports through which most of the air flows to the outside. It is a more particular feature of this embodiment that the isolator has an air lock that can be severed from the bag portion of the isolator when the surgery is completed to protect the instruments that have been used during the surgery from the air in the operating room so that the instruments will be readily available, if needed, during the period between the completion of the surgery and the moving of the patient from the operating room.

The described embodiments of this invention also provide an arrangement for filtering the air being pumped into the bag to decrease the possibility of contaminating the sterile isolator bag when the bag is connected to the blower supplying the air to the bag. More particularly there will be described a blower-air filter assembly for providing filtered air under pressure to the bag of the isolator in which any leakage will be of filtered air back to the suction side of the blower to keep non-filtered air from entering the bag. In one specific arrangement, the bag that is inflated with sterile air from the blower-filter assembly that passes through another filter located in the sterile air duct between the blower-filter assembly and the bag so that the connection between the sterile air duct and the blower-filter assembly is made upstream from this filter to entrap any contaminants that are introduced into the air duct when the connection is made.

In the embodiments to be described a portion of the lower side of the inflatable bag is made of an elastic material through which a body member upon which a surgical procedure is to be performed, can be pulled into the work space in the bag through an incision in the elastic material that is smaller than the member to cause the stretched elastic material to tightly surround the member and isolate the body member from the environment outside the work space.

Mention has been made of the enclosures of the prior art being provided with gloves to allow manipulation within the enclosure. The gloves may be fixed at the end of the sleeves. In the embodiment of the present invention to be described a special form of sleeve is employed which enables the surgeon, for example, to gain access to the interior of the inflatable bag.

As well as finding application to the problems of infections in surgical procedures, the isolator of this invention also has utility in industry. It can replace the "clean rooms" now used to provide a contamination-free atmosphere in which equipment is assembled that is especially sensitive to environmental contamination, such as dust. A typical clean room has an atmospheric-control system that rigidly controls temperature and humidity and bars entrance, by means of filters, of all but the tiniest mote of dust. Walls and ceilings are of one-piece plastic with no cracks where dust might collect and are washed and vacuumed daily. Maintenance cannot be done within the room: plumbing, wiring, and lighting are so arranged that maintenance can be handled in crawl spaces above the ceiling. The room has no sharp corners; they are rounded off to forestall dust collection.

Before entering, workers don special clean suits, including head covering and boots, and pass under an "air shower" that removes all loose particles of matters. The parts that make up the assembly are thoroughly cleaned and polished before delivery to the clean room, which they enter through an air lock.

By employing an isolator incorporating the teachings of this invention, much of the elaborate equipment, special room design, and special clean suits can be eliminated greatly reducing the cost of a clean room operation.

Broadly stated, the present invention provides an isolator for providing a contamination-free atmosphere in which a surgical procedure can be performed or equipment that is sensitive to environmental contamination can be assembled, the isolator comprising a bag having bottom, side and end walls of flexible impervious material, an inlet and an outlet and blower means for circulating filtered air in the bag from the inlet to the outlet, characterised in that:
the bag has an upper wall that is supported above said bottom wall solely by the inflation of the bag by the air pressure established therein;
said inlet and outlet are on opposite sides of a zone within the inflated bag at which a surgical procedure or equipment assembly is to be performed;
said upper wall comprises a window of a relatively-stiff, optically transparent material that provides a clear view of said zone for more than one person and that assumes a stable viewing position supported solely by the air pressure established in the bag; and
said blower means is operable to provide a flow through the bag at a rate that maintains the bag inflated to support said upper wall and that is at least twice the bag volume per minute.

The window referred to above is preferably of a convex shape in the transverse direction of the bag between said side walls and it is straight in the direction between said end walls.

In the preferred embodiments of the invention a plurality of open-ended sleeves are attached to the bag, through which the hands and arms of workers can extend into the interior of the bag, each sleeve having elastic material encircling the sleeve adjacent its outer end to hold the sleeve tightly around an arm extending through the sleeve to reduce the flow of air from the bag between the arm and the sleeve and to cause the sleeve to intussuscept as required to allow the arm to move freely into and out of the bag.

The invention and its preferred practice will now be described with reference to embodiments of it already discussed above and illustrated in the accompanying drawings in which:
Figure 1 is a side view, in elevation, of one embodiment of the isolator of this invention inflated and in position on a patient shown in dotted lines;
Figure 2 is a top view of the isolator of Figure 1;
Figure 3 is a sectional view taken along line 3-3 of Figure 1;
Figure 4 is a top view of a surgeon with his arms extending through two of the sleeves of the isolator showing the intussusceptive action of the sleeves as the surgeon's arms move into the isolator;
Figure 5 is a side view, in elevation, of the isolator showing an alternate arrangement for positioning the filter used to filter the air entering the isolator;
Figure 6 is a side view in elevation of an alternate embodiment of the isolator of this invention;
Figure 7 is a plan view of the isolator of Figure 6;
Figure 8 is a sectional view taken along line 8-8 of Figure 6;
Figure 9 is a sectional view, on an enlarged scale, taken along line 9-9 of Figure 7;
Figure 10 is a view in elevation looking in the direction of arrows 10-10 of Figure 9; and
Figure 11 is a sectional view on an enlarged scale, taken along line 10-10 of Figure 7.

The isolator shown in the drawings is described below as it is used in a surgical procedure. As explained above, it can be used as a clean room for industrial purposes equally as well.

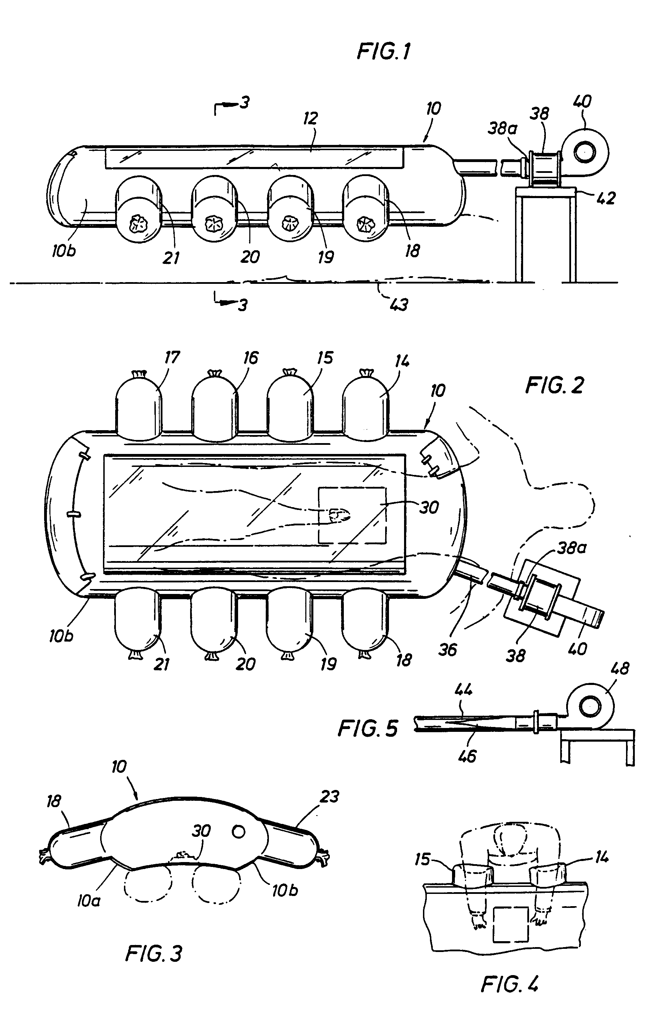

The isolator shown in Figures 1-5 and generally indicated by the number 10, is essentially a bag that is inflated in use. It is made of materials through which disease organisms cannot pass. Most of the bag, in the preferred embodiment, is made of a clear, flexible acrylic resin plastic except for optical window 12 in the top portion of the isolator and a portion of the bottom side of the isolator, where a special material is provided for certain surgical procedures that will be described below.

Window 12 is made out of a relatively stiff, optically clear, plastic material, such as the thermoplastic carbonate-linked polymer produced by reacting bisphenol A and phosgene and sold under the trademark "Lexan" by the General Electric Co. It provides the surgical team with a clear, undistorted, view of the surgical field. In addition, the window, being relatively stiff compared to the acrylic material used for the rest of the bag, will cause the inflated bag to assume a more or less elliptical shape in cross-section as shown in Figure 3. In the embodiment shown in Figures 1-5, the window is substantially straight along the longitudinal axis of the bag and slightly convex along the transverse axis. It is also a stable window that will assume a position relative to the bottom of the bag and substantially maintain that position even though the pressure in the bag may change slightly during the time it is in use. Further, it will not undulate or balloon under the pressure, which could distort the view through the window.

Side walls 10a and 10b of the isolator are provided with openings that connect to open ended sleeves 14-21 attached to the side wall of the isolator. In this embodiment, four sleeves are provided on each side of the isolator. The sleeves are open ended, but are held substantially closed, when not in use, by rubberized elastic attached to the sleeves adjacent the ends. In use, the surgeon will extend his arms through the openings in, for example, sleeves 14 and 15, as they are held open by an assistant, until the elastic band of the sleeves is somewhere above his elbow. Then as the surgeon extends his arms into the working space between the bottom of the isolator and window 12, the sleeves will turn inwardly on themselves or intussuscept as required to allow the arms to which they are attached to move freely into the isolator. In Figure 5, for example, the surgeon has his arms sufficiently inside the isolator to perform his surgery, yet sleeves 14 and 15 are outside of the working space of the isolator so as not to interfere with the movement of the surgeon's hands or arms or interfere with his vision of the surgical field.

In this arrangement, the surgeon is gloved outside the isolator. If he needs to change gloves during the surgery, he can simply pull his arms out of sleeves 14 and 15, be regloved, and reinsert his hands and arms back into the isolator through sleeves 14 and 16. This can be done quickly and easily.

Further, if for some reason, it is necessary to remove the isolator before the surgery is completed, the surgeon can remove his arms from the sleeves, remove the isolator, and immediately continue without having to be regloved as is the case with the prior art isolators. Some air leaks out through sleeves 14 and 15 both when the surgeon has his arms in the sleeves and also when they are not in use. This positive flow of air along the sleeves of the surgeon and out through the opening end of the sleeves through which the surgeon's arms extend, prevents disease organisms from the surgeon from entering the isolator to contaminate and infect the surgical wound.

A portion of the bottom of the isolator bag is made out of a surgical drape of resilient material. This allows a body member, upon which a surgical procedure is to be performed, to be pulled into the work space of the isolator through an incision in the drape that is smaller than the body member. The stretched elastic material tightly engages the member to reduce the chances of disease organisms from entering the work space from the outside. Any air that leaks through the opening in the drape will tend to carry such organisms away from the work space. This feature, of course, is advantageous where the body member is of such shape add size that it can be pulled into the work space conveniently so as to isolate it from the outside environment and from the body of the patient itself. For example, a foot, a knee, a whole leg, or a portion thereof, hands, arms, head, and genitalia are examples of body members that could be so handled.

In Figure 2, drape portion 30 is positioned so that the patient's genitalia can be pulled through an incision in the drape and isolated from the outside during a surgical procedure, such as the implantation of a penile prosthesis.

Air conduit 36 is integrally attached to the isolator and sterilized along with the isolator before it is placed in use. In the operation room, the end of conduit 36 is attached to filter 38 connected to the outlet of blower 40. Filter 38 is of the type that will filter out disease organisms to prevent them from entering the isolator. The filter and blower are supported on table 42 located adjacent operating table 43.

In most cases, the outlet side of filter 38 will not be as sterile as is desired for use with the isolator. Even if sterile, someone has to make a connection between conduit 36 and the outlet of the filter and this can introduce disease organisms into the conduit when the connection is being made. The connection usually consists of slipping the end of conduit 36 over outlet to 38a and clamping or taping the conduit in place.

A conduit 44 is provided that has conical filter 46 installed in the conduit downstream from its end, as shown in Figure 5. Filter 46 will prevent any disease organisms that are introduced into the conduit, while it is being connected to the blower, from reaching the isolator. Filter 38, described above, is classified as a high efficiency particle air (HEPA) filter. Conically shaped filter 46, in the embodiment shown in Figure 4, is made of thin plastic membrane like material having a porosity of several million pores per square inch that can filter bacteria out of the moving air stream. One such material is marketed by E.I. duPont de Nemours & Co. under the trademark "REMAY", Style 2016, and is made of a spun bound polyester.

One end wall of the isolator includes rather large flap 50 that is held in the closed position by tape. Instruments and the like are moved into and out of the isolator through this flap. Suction tubes, cautery, and the like can be inserted through smaller flap 52 at the other end. Air continuously leaks out of the isolator through the flaps and the sleeves. This is desirable since there should be a continuous flow of sterile air over the surgical wound, not a blast of air, of course, but steady movement. The elastic holding the sleeves closed can function to regulate pressure in the isolator. As it increases, it will open the sleeves letting out more air and vice versa.

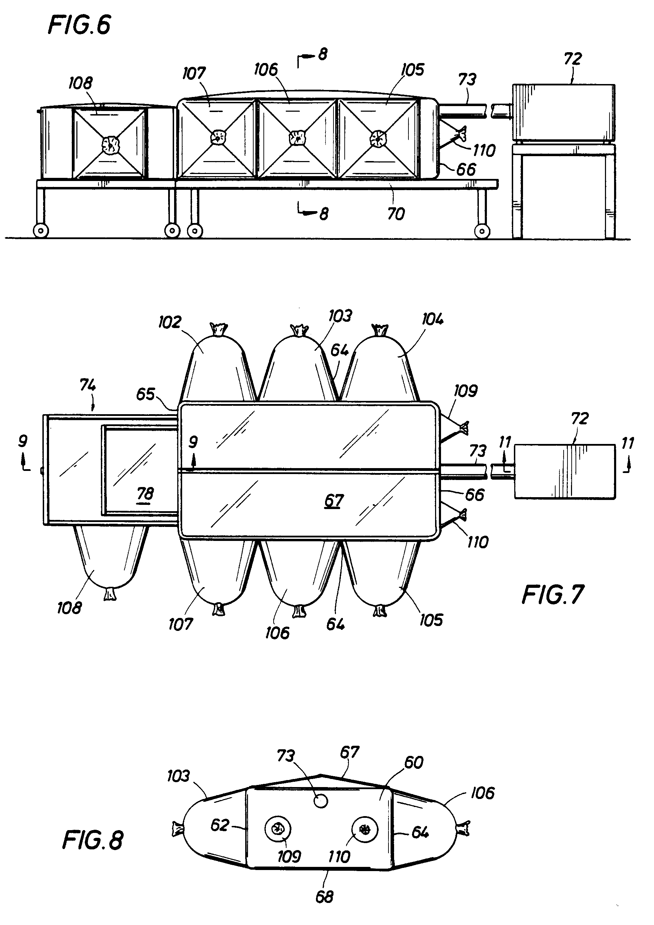

An alternate embodiment of the surgical isolation system of this invention is shown in Figures 6-11. It includes inflatable bag or bubble 60, which is generally rectangular in cross-section, having side walls 62 and 64, end walls 65 and 66, top 67, and bottom 68. The bag in Figure 6 is shown inflated and resting on operating table 70. There is a patient between the bag and the operating table, but for the sake of clarity, the patient is not shown. It is understood that the bag is resting on top of the patient although some part of the patient may be pulled inside of the bag, if the surgery is on a portion of the body that can be pulled inside the bag. Filtered air is supplied to the bag from blower-filter assembly 72 through duct 73 that is connected to an opening in end wall 66. Air travels the length of the bag and is exhausted through exhaust ports in end wall 65.

It is very important that the air in the bag be changed continuously. In one embodiment of the invention, the blower-filter assembly provided ten times the volume of the bag every minute. To maintain the bag properly inflated with an adequate flow of air through the bag, a volume of air equal to at least double the volume of the bag should be supplied to the bag every minute.

In the embodiment shown, air lock 74 is attached to end 65 of the bubble. The air lock is used to pass instruments, materials, and prostheses into and out of the bubble. It also serves as the conduit through which most of the air is exhausted from the bag.

In the embodiment shown, the air lock includes a rigid framework of angle iron connected to form rectangular end frames 75 and 76 that are held upright by struts 77 extending between the corners of the end frames. The outside of the framework is covered by the same clear, flexible plastic used for the bag except for window 78 in the top that is made of the same rigid optically transparent material used for window 67 in the top of the bag.

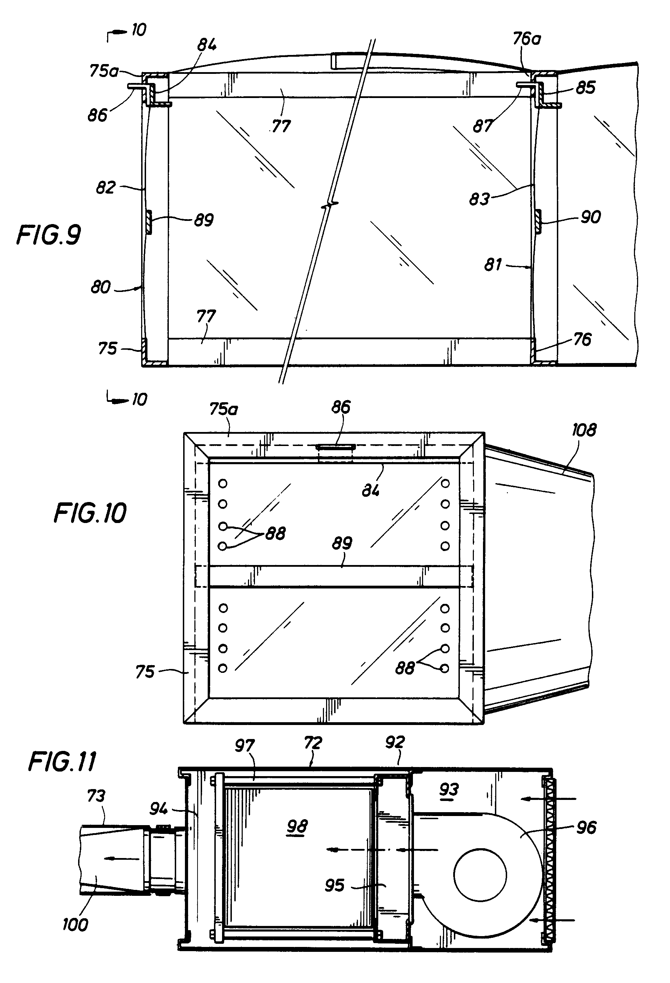

End frame 76 is connected to an opening in end wall 65 of the bag. To function as an air lock, doors or the like are located at each end, one to allow material to be moved into and out of the air lock from the outside. The other to allow material to be moved into and out of the air lock from the bag. In the embodiment shown, these door functions are provided by end flaps 80 and 81. The flaps include rectangular sheets 82 and 83 of clear, flexible plastic that are connected along the lower edge to the bottom of the end frames. The opposite sides of the flaps are connected to rigid support members 84 and 85. The flaps are held in position closing the air lock by L-shaped latch members 86 and 87 attached to the middle of the flaps opposite support members 84 and 85 and extend through slots in upper end frame members 75a and 76a, as shown in Figure 9. Either end of the air lock can be opened by removing the latch member from the slot, allowing the flap to collapse to the bottom of the air lock.

Openings 88 are provided in the flaps to serve as exhaust ports through which air can flow out of the bag through the air lock into the operating room. It is intended for most of the air exhausted from the bag to flow through these openings. Therefore, the size and member of openings are chosen to allow the air to flow out of the bag at about the rate that air is pumped into the bag with the pressure drop across the flaps being sufficient to maintain the desired inflation pressure in the bag. Due to the pressure drop across the flaps, rigid members 89 and 90 are attached to the flaps across the middle to engage the end frames and support the flaps.

Blower-filter assembly 72 includes housing 92 that is divided into blower compartment 93 and filter compartment 94 by plenum 95. In this embodiment, the plenum is a rectangular box having openings on opposite sides. Mounted on one side to pump air into the plenum is blower 96. Mounted on the other by bolts 97 is HEPA filter 98. Mounting flanges 99 support the blower, filter, and plenum in the housing. Appropriate seals are located between the blower and the plenum and the plenum and the HEPA filter to keep unfiltered air from entering filter compartment 94 and air duct 73. Should any of the seals leak, however, with the arrangement described above, since the pressure in filter compartment 94 is greater than the pressure outside housing 72 and the pressure in blower compartment 93, filtered air will leak out of filter compartment 94, but unfiltered air cannot enter the compartment and contaminate the air entering the bag.

Conical filter 100 serves the same purpose as conical filter 46 described above in connection with Figure 5.

In this embodiment, the bag has three sleeves 102, 103, and 104 on one side and three sleeves 105, 106, and 107 on the other side. Air lock 74 has one sleeve 108. These function in the same manner as the sleeves described above in connection with the embodiment shown in Figures 1-5. They are constructed differently, however, being formed of four, flat triangular pieces of plastic connected at their edges to each other and the side wall of the bag.

Smaller sleeves 109 and 110 in end wall 66 allow the passage of suction and electrocautery connections to the outside of the bag. The elastic around the openings in these sleeves provides a substantially airtight seal around the arms of the surgical team and the electrical cables and pneumatic hoses extending through the sleeves.

In operation, the air lock can be supported by a separate table, such as table 112. This allows the air lock to be disconnected from the bag after the surgery is completed and the surgical instruments have been moved back into the air lock. It can be disconnected simply by cutting it from wall 65 leaving it intact on table 112. The bag can then be removed and the patient prepared for removal from the operating room. While this is going on, the instruments will be somewhat protected from the air in the operating room and be available should the need arise.

## Claims

1. An isolator for providing a contamination-free atmosphere in which a surgical procedure can be performed or equipment that is sensitive to environmental contamination can be assembled, the isolator comprising a bag (10, 60) having bottom, side and end walls of flexible impervious material, an inlet (36) and an outlet (50) and blower means (38, 40) for circulating filtered air in the bag from the inlet (36) to the outlet (50), characterised in that:
the bag has an upper wall that is supported above said bottom wall solely by the inflation of the bag by the air pressure established therein;
said inlet and outlet are on opposite sides of a zone (30) within the inflated bag (10, 60) at which a surgical procedure or equipment assembly is to be performed;
said upper wall comprises a window (12) of a relatively stiff, optically transparent material that provides a clear view of said zone (30) for more than one person and that assumes a stable viewing position supported solely by the air pressure established in the bag; and
said blower means (38, 40) is operable to provide a flow through the bag at a rate that maintains the bag inflated to support said upper wall and that is at least twice the bag volume per minute.

2. An isolator according to Claim 1 in which said window (12) is of a convex shape in the transverse direction of the bag between said side walls.

3. An isolator according to Claim 1 in which said window (12) is straight in the direction between said end walls and convex in a direction between said side walls.

4. An isolator according to Claim 1 or 2 in which a plurality of sleeves (14-21) for gaining access to the interior of said bag (10) are located in the said side walls of said bag.

5. An isolator according to Claim 1 in which a plurality of open-ended sleeves (14-21) are attached to the bag (10), through which the hands and arms of workers can extend into the interior of the bag, each sleeve having elastic material encircling the sleeve adjacent its outer end to hold the sleeve tightly around an arm extending through the sleeve to reduce the flow of air from the bag between the arm and the sleeve and to cause the sleeve to intussuscept as required to allow the arm to move freely into and out of the bag.

6. An isolator according to Claim 4 in which said sleeves (14-21) are open-ended to allow the hands and arms of workers to extend into the bag, each sleeve having elastic material encircling the sleeve adjacent its outer end to hold the sleeve tightly around an arm extending through the sleeve to reduce the flow of air from the bag between the arm and the sleeve and to cause the sleeve to intussuscept as required to allow the arm to move freely into and out of the bag.

7. An isolator as claimed in any preceding claim in which said inlet and outlet are located in respective end walls of the bag (10).

8. An isolator as claimed in Claim 7 comprising an air lock (74) through which surgical instruments and other equipment is movable into the bag, the air lock (74) being attached to the end wall of the bag in which said outlet is located.

9. An isolator as claimed in Claim 8 in which the air lock (74) comprises an open-ended chamber having one end attached to the end wall of the bag opposite the end wall through which air is supplied to the bag, an opening in the end wall through which instruments and other equipment can be moved back and forth between the bag and the chamber, doors at each end of the chamber for opening and closing the open ends of the chamber, and in which openings in the doors provide exhaust ports through which air is vented from the bag through the chamber.

10. An isolator as claimed in any preceding claim in which a portion (30) of the bottom of the bag is made of surgical drape material for placing over the patient where the incision is to be made during a surgical procedure.

11. The isolator as claimed in any one of Claims 1 to 9 in which a portion (30) of the bottom of the bag is made of elastic surgical drape material to allow a body member upon which a surgical procedure is to be performed, to be pulled into the work space through an incision in the drape that is smaller than the member to cause the stretched elastic material to tightly surround the member and reduce the chances of disease organisms entering the work space from the outside.

12. An isolator as claimed in any preceding claim in which the blower means includes a housing (92) having an interior wall that divides the housing into first (93) and second (94) chambers, said first chamber having an opening through which air can enter the first chamber, an air filter located in the opening to filter larger airborne contaminants including larger bacteria, dust particles, and the like from the air entering the first chamber, a blower (96) located in the first chamber (93) to pump air from the first chamber into the second chamber (94), a plenum (95) in the second chamber into which the air from the blower is discharged, a second filter (48) attached to the plenum (95) to filter the remaining contaminants from the air, said plenum (95) and second filter (98) being spaced from the outside walls of the second chamber (94) so that the pressure in the second chamber (94) will be higher than the pressure in the first chamber (93) to ensure any leakage between the chambers will be filtered air flowing from the second (94) to the first (93) chamber, and means (73) connecting the second chamber to the bag.

13. An isolator as claimed in any one of Claims 1 to 11 in which the blower means (38, 40) includes a sterile air duct (44) through which air is supplied to the interior of the bag (10) at least slightly higher than atmospheric pressure by a blower (48) and a sterile filter (46) located in the air duct (44) downstream from the end of the duct to be connected to the blower (48) so that the connection between the sterile air duct (44) and the blower (48) is made upstream from the sterile filter (46) to allow any non-sterile particles or disease organisms that are introduced into the air duct (44) when the connection is made or in the air supplied by the blower (48) to be trapped by the filter (46) before they enter the bag (40).

## Patentansprüche

1. Isolator zur Schaffung einer vor Verunreinigungen freien Atmosphäre, in der sich ein operativer Eingriff durchführen läßt oder Geräte, die gegen Verunreinigungen aus der Umgebung empfindlich sind, zusammenbauen lassen, wobei der Isolator eine Hülle (10, 60) mit Boden-, Seiten- und Endwänden aus flexiblem undurchlässigen Material, einen Einlaß (36) und einen Auslaß (50) sowie eine Gebläseeinrichtung (38, 40) zum Zirkulierenlassen gefilteter Luft in der Hülle von dem Einlaß (36) zu dem Auslaß (50) aufweist,
dadurch gekennzeichnet,
daß die Hülle eine obere Wand aufweist, die lediglich durch das Aufblasen der Hülle durch den darin hergestellten Luftdruck über der Bodenwand getragen ist;
daß der Einlaß und der Auslaß auf einander gegenüberliegenden Seiten einer innerhalb der aufgeblasenen Hülle (10, 60) befindlichen Zone (30) liegen, in der ein operativer Eingriff oder eine Gerätemontage durchzuführen ist;
daß die obere Wand ein Fenster (12) aus relativ steifem, optisch transparenten Material aufweist, das mehr als einer Person eine klare Sicht der Zone (30) ermöglicht und eine stabile Betrachtungsposition einnimmt, in der es ausschließlich durch den in der Hülle hergestellten Luftdruck getragen ist; und
daß die Gebläseeinrichtung (38, 40) zur Schaffung einer Strömungsrate durch die Hülle betreibbar ist, die die Hülle zum Tragen der oberen Wand aufgeblasen hält und wenigstens das Doppelte des Hüllenvolumens pro Minute beträgt.

2. Isolator nach Anspruch 1,
wobei das Fenster (12) eine konvexe Form in Querrichtung der Hülle zwischen den Seitenwänden aufweist.

3. Isolator nach Anspruch 1,
wobei das Fenster (12) in Richtung zwischen den Endwänden gerade ist und in Richtung zwischen den Seitenwänden konvex ist.

4. Isolator nach Anspruch 1 oder 2,
wobei eine Mehrzahl von Ärmeln (14-21) zur Ermöglichung eines Zugangs zum Inneren der Hülle (10) in den genannten Seitenwänden der Hülle vorgesehen ist.

5. Isolator nach Anspruch 1,
wobei eine Mehrzahl offenendiger Ärmel (14-21) an der Hülle (10) angebracht ist, durch die sich die Hände und Arme von Arbeitern ins Innere der Hülle hindurchführen lassen, wobei jeder Ärmel elastisches Material aufweist, das den Ärmel in der Nähe seines äußeren Endes umgibt, um den Ärmel fest um einen sich durch den Ärmel hindurcherstreckenden Arm herumzuhalten, um den Luftstrom aus der Hülle heraus zwischen dem Arm und dem Ärmel zu reduzieren und den Ärmel zum nach innen Stülpen zu veranlassen, wie dies zur Ermöglichung einer freien Bewegung des Arms in die Hülle hinein und aus dieser heraus erforderlich ist.

6. Isolator nach Anspruch 4,
wobei die Ärmel (14-21) offenendig sind, so daß sich die Hände und Arme von Arbeitern in die Hülle hineinerstrecken könnten, wobei jeder Ärmel elastisches Material aufweist, das den Ärmel in der Nähe seines äußeren Endes umgibt, um den Ärmel fest um einen sich durch den Ärmel hindurcherstreckenden Arm zu halten, um den Luftstrom aus der Hülle heraus zwischen dem Arm und dem Ärmel zu reduzieren sowie ein nach innen Stülpen des Ärmels zu verursachen, wie dies zur Ermöglichung einer freien Bewegung des Arms in die Hülle hinein und aus dieser heraus erforderlich ist.

7. Isolator nach einem der vorausgehenden Ansprüche,
wobei sich der Einlaß und der Auslaß in den jeweiligen Endwänden der Hülle (10) befinden.

8. Isolator nach Anspruch 7,
mit einer Luftschleuse (74), durch die hindurch sich Operationsinstrumente und andere Geräte in die Hülle hineinbewegen lassen, wobei die Luftschleuse (74) an derjenigen Endwand der Hülle angebracht ist, in der sich der Auslaß befindet.

9. Isolator nach Anspruch 8,
wobei die Luftschleuse (74) eine offenendige Kammer aufweist, deren eines Ende an derjenigen Endwand der Hülle angebracht ist, die der Endwand, durch die Luft in die Hülle eingeleitet wird, gegenüberliegt, und die eine Öffnung in der Endwand, durch die hindurch sich Instrumente und andere Geräte zwischen der Hülle und der Kammer vor und zurückbewegen lassen, sowie Türen an jedem Ende der Kammer zum Öffnen und Schließen der offenen Enden der Kammer aufweist, und wobei Öffnungen in den Türen Austrittsöffnungen schaffen, durch die Luft aus der Hülle durch die Kammer hindurch ausgeleitet wird.

10. Isolator nach einem der vorausgehenden Ansprüche,
wobei dort, wo während eines operativen Eingriffs ein Einschnitt vorzunehmen ist, ein Bereich (30) des Bodens der Hülle aus Operationsdeckenmaterial zum Plazieren über dem Patienten hergestellt ist.

11. Isolator nach einem der Ansprüche 1 bis 9,
wobei ein Bereich (30) des Bodens der Hülle aus elastischem Operationsdeckenmaterial gebildet ist, so daß sich ein Körperteil, an dem ein operativer Eingriff durchzuführen ist, durch einen Einschnitt in der Decke, der kleiner ist als das Körperteil, in den Arbeitsraum hineinziehen läßt, so daß das gespannte elastische Material das Körperteil fest umgibt und die Möglichkeiten für ein Eindringen von Krankheitserregern in den Arbeitsraum von außen her reduziert sind.

12. Isolator nach einem der vorausgehenden Ansprüche,
wobei die Gebläseeinrichtung ein Gehäuse (92) aufweist, das eine das Gehäuse in eine erste (93) und eine zweite (94) Kammer unterteilende Innenwand aufweist, wobei die erste Kammer eine Öffnung besitzt, durch die Luft in die erste Kammer eintreten kann, wobei sich in der Öffnung ein Luftfilter befindet zum Herausflltern größerer in der Luft mitgeführter Verunreinigungen, die größere Bakterien, Staubpartikel und dergleichen beinhalten, aus der in die erste Kammer eintretenden Luft, wobei sich in der ersten Kammer (93) ein Gebläse (96) befindet, um Luft von der ersten Kammer in die zweite Kammer (94) zu pumpen, wobei in der zweiten Kammer eine Plenumkammer (95) vorgesehen ist, in die die Luft von dem Gebläse abgegeben wird, wobei an der Plenumkammer (95) ein zweiter Filter (48) zum Herausfiltern der übrigen Verunreinigungen aus der Luft angebracht ist, wobei die Plenumkammer (95) und der zweite Filter (98) von den Außenwänden der zweiten Kammer (94) beabstandet sind, so daß der Druck in der zweiten Kammer (94) höher ist als der Druck in der ersten Kammer (93), um sicherzustellen, daß jegliches Leck zwischen den Kammern zu einem Strom gefilterter Luft von der zweiten (94) in die erste (93) Kammer führt, und wobei eine die zweite Kammer mit der Hülle verbindende Einrichtung (73) vorgesehen ist.

13. Isolator nach einem der Ansprüche 1 bis 11,
wobei die Gebläseeinrichtung (38, 40) einen sterilen Luftkanal (44) aufweist, durch den hindurch Luft in das Innere der Hülle (10) mit einem wenigstens geringfügig höheren Druck als Atmosphärendruck durch ein Gebläse (48) und einen sterilen Filter (46) eingeleitet wird, der in dem Luftkanal (44) stromab von dem mit dem Gebläse (48) zu verbindenden Ende des Kanals angeordnet ist, so daß die Verbindung zwischen dem sterilen Luftkanal (44) und dem Gebläse (48) stromauf von dem sterilen Filter (46) erfolgt, so daß sich jegliche nicht-sterile Teilchen oder Krankheitserreger, die bei der Herstellung der Verbindung in den Luftkanal (44) eingebracht werden oder in der von dem Gebläse (48) abgegebenen Luft enthalten sind, vor dem Eintritt in die Hülle (40) durch den Filter (46) auffangen lassen.

## Revendications

1. Dispositif d'isolation assurant une atmosphère non contaminée dans laquelle on peut pratiquer une intervention chirurgicale ou dans laquelle on peut assembler du matériel naturellement sensible à la contamination environnante, ce dispositif d'isolation comprenant un sac (10, 60) muni d'un fond, de parois et d'extrémités réalisés dans un matériau souple impénétrable, d'une entrée (36), d'une sortie (50) et de moyens de soufflerie (38, 40) pour faire circuler l'air filtré dans le sac depuis l'entrée (36) jusqu'à la sortie (50), caractérisé en ce que :
le sac a une paroi supérieure maintenue au-dessus de la paroi inférieure uniquement par le gonflement du sac par la pression d'air qui y est établie;
l'entrée et la sortie sont situées sur les côtés opposés d'une zone (30) située à l'intérieur du sac gonflé (10, 60), dans laquelle on va pratiquer une intervention chirurgicale ou assembler du matériel;
la partie supérieure comprend une lucarne (12) en matériau relativement rigide et optiquement transparent fournissant une vue dégagée de la zone (30) pour plus d'une personne et assurant une position stable de vision grâce à l'unique apport de pression d'air établie dans le sac; et
les moyens de soufflerie (38, 40) sont mis en service pour fournir un flux traversant le sac à un débit maintenant le sac gonflé pour supporter sa partie supérieure, lequel flux est au moins égal par minute à deux fois le volume du sac.

2. Dispositif d'isolation selon la revendication 1 dans lequel la lucarne (12) a une forme convexe dans le plan transversal du sac entre ses parois latérales.

3. Dispositif d'isolation selon la revendication 1 dans lequel la lucarne (12) est droite dans le plan situé entre les parois d'extrémité et convexe dans le plan situé entre les parois latérales.

4. Dispositif d'isolation selon la revendication 1 ou 2, dans lequel une pluralité de manchons (14-21) permettant l'accès à l'intérieur du sac (10) sont placés sur les côtés du sac.

5. Dispositif d'isolation selon la revendication 1 dans lequel une pluralité de manchons à l'extrémité ouverte (14-21) sont attachés au sac (10), au travers desquels les mains et les bras des travailleurs peuvent s'avancer dans l'intérieur du sac, chaque manchon étant muni d'un matériau élastique qui l'enserre près de son extrémité extérieure pour maintenir fermement le manchon autour d'un bras passé dans celui-ci afin de réduire entre le bras et ce manchon le flux d'air venant du sac et permettre au manchon l'invagination requise autorisant le bras à se déplacer librement à l'intérieur du sac et à en sortir.

6. Dispositif d'isolation selon la revendication 4 dans lequel l'extrémité des manchons (14-21) est ouverte pour permettre aux mains et aux bras des travailleurs de s'avancer dans le sac, chaque manchon étant pourvu d'un matériau élastique qui l'enserre près de son extrémité extérieure pour maintenir fermement le manchon autour d'un bras passé dans celui-ci afin de réduire entre le bras et ce manchon le flux d'air venant du sac et permettre au manchon l'invagination requise autorisant le bras à se déplacer librement à l'intérieur du sac et à en sortir.

7. Dispositif d'isolation selon l'une quelconque des revendications précédentes dans lequel une entrée et une sortie sont placées aux extrémités respectives du sac (10).

8. Dispositif selon la revendication 7 comprenant un sas à air (74) au travers duquel on fait passer dans le sac les instruments chirurgicaux et autres matériels, ce sas à air (74) étant fixé à l'extrémité du sac dans laquelle la sortie est placée.

9. Dispositif d'isolation selon la revendication 8 dans lequel le sas à air (74) comprend une chambre ouverte dont une extrémité est fixée à l'extrémité de la paroi du sac opposée à l'extrémité par de laquelle le sac est alimenté en air, une ouverture dans l'extrémité entre le sac et la chambre au travers de laquelle les instruments et autres matériels peuvent être retirés ou réintroduits, des portes à chaque extrémité de la chambre pour ouvrir ou fermer ses ouvertures, ces portes ayant des orifices de pompage au travers desquels l'air est soutiré.

10. Dispositif d'isolation selon l'une quelconque des revendications précédentes dans lequel une partie (30) du fond du sac est réalisée dans un drapé chirurgical que l'on place au-dessus du patient à l'endroit qu'il faut inciser pendant l'intervention chirurgicale.

11. Dispositif d'isolation selon l'une quelconque des revendications 1 à 9 dans lequel une partie (30) du fond du sac est réalisée dans un drapé chirurgical pour permettre à un membre d'un corps sur lequel on va pratiquer une intervention chirurgicale, d'être tiré à l'intérieur de l'espace de travail au travers d'une incision dans le drapé qui est plus petite que le membre pour faire en sorte que le matériau élastique étiré enserre fermement le membre et réduise les possibilités pour des agents infectieux de pénétrer de l'extérieur dans l'espace de travail.

12. Dispositif d'isolation selon l'une quelconque des revendications précédentes dans lequel une soufflerie comprend un coffrage (92) ayant une paroi intérieure qui divise ledit coffrage en une première (93) et une seconde (94) chambres, la première chambre ayant une ouverture au travers de laquelle l'air peut entrer, un filtre à air placé à l'ouverture pour filtrer à partir de l'air entrant dans la première chambre les plus grosses substances contaminantes aoportées par celui-ci comprenant les plus grosses bactéries, des particules de poussière et autres choses semblables, une soufflerie (96) située dans la première chambre (93) pour pomper l'air de la première chambre vers la deuxième chambre (94), un plenum (95) dans la seconde chambre dans lequel l'air en provenance de la soufflerie est collecté, un second filtre (98) fixé au plenum (95) pour filtrer les substances contaminantes restant dans l'air, ce plenum (95) et le second filtre (98) étant espacés des parois extérieures de la seconde chambre (94) de sorte que la pression de la seconde chambre (94) soit plus élevée que celle de la première chambre (93) afin que toute fuite d'air entre les chambres soit filtrée, de l'air fluant de la seconde (94) chambre vers la première (93), et des moyens (73) pour connecter la seconde chambre au sac.

13. Dispositif d'isolation selon l'une quelconque des revendications 1 à 11 dans lequel les moyens de soufflerie (38, 40) comprennent un conduit d'air stérile (44) au travers duquel l'air est alimenté dans l'intérieur du sac (10) à une pression légèrement supérieure à la pression atmosphérique par une soufflerie (48) et un filtre stérile (46) situé dans le conduit d'air (44) en aval de l'extrémité du conduit qui doit être connecté à la soufflerie (48) de manière que la connexion entre le conduit d'air stérile (44) et la soufflerie (48) se fasse en amont du filtre stérile (46) pour permettre à toute particule non-stérile ou à tout agent infectieux introduit, lorsque la connexion est faite, dans le conduit d'air (44) ou dans l'air alimenté par la soufflerie (48), d'être capté par le filtre (46) avant d'entrer dans le sac (10).
